(19) Europäisches Patentamt
European Patent Office
Office européen des brevets

(11) EP 2 599 780 A1

(12) EUROPEAN PATENT APPLICATION
published in accordance with Art. 153(4) EPC

(43) Date of publication:
**05.06.2013 Bulletin 2013/23**

(21) Application number: **11812068.2**

(22) Date of filing: **27.07.2011**

(51) Int Cl.:
***C07D 471/04*** (2006.01) ***H01L 51/42*** (2006.01)

(86) International application number:
**PCT/JP2011/004246**

(87) International publication number:
**WO 2012/014466 (02.02.2012 Gazette 2012/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.07.2010 JP 2010168789**

(71) Applicant: **Idemitsu Kosan Co., Ltd.**
**Chiyoda-ku**
**Tokyo 100-8321 (JP)**

(72) Inventors:
- **YASUKAWA, Keiichi**
**Sodegaura-shi**
**Chiba 299-0293 (JP)**
- **MAEDA, Ryoji**
**Sodegaura-shi**
**Chiba 299-0293 (JP)**
- **TOKAILIN, Hiroshi**
**Sodegaura-shi**
**Chiba 299-0293 (JP)**

(74) Representative: **Gille Hrabal**
**Brucknerstrasse 20**
**40593 Düsseldorf (DE)**

# (54) PHENANTHROLINE COMPOUND, ELECTRON TRANSPORT MATERIAL OBTAINED FROM SAID COMPOUND, AND ORGANIC THIN-FILM PHOTOVOLTAIC CELL COMPRISING SAID COMPOUND

(57) A compound represented by the following formula (1), wherein L is a single bond or a divalent group, and is bonded at any one of the 6th, 8th and the 9th positions indicated by * of 1,10-phenanthroline; Rg is a substituted or unsubstituted benzene ring or a substituted or unsubstituted naphthalene ring; and X is an oxygen atom or N-$R^4$.

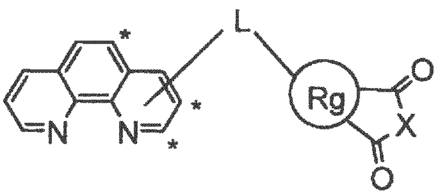

(1)

EP 2 599 780 A1

## Description

Technical field

The invention relates to a novel phenanthroline compound, an electron-transporting material comprising the same, a material for an organic thin film solar cell and an organic thin film solar cell using the same and a stacked organic thin film solar cell.

Background art

Baked by the shortage of fossil fuel or global warming, a solar cell has recently attracted attention as the source of clean energy, and research and development on a solar cell has been aggressively made. Heretofore, silicon-based solar cells using monoaystalline Si, polycrystalline Si, amorphous Si, or the like have been put into practical use. Since the problem such as expensiveness or the shortage or the like of raw material Si has come up to the surface, there has been an increasing demand for a next-generation solar cell.
Under such circumstances, due to inexpensiveness, and the absence of a material shortage risk, an organic solar cell has attracted attention as the next generation solar cell which takes the place of a silicon-based solar cell.
An organic solar cell is basically composed of an n layer which transfers electrons and a p layer which transfers holes.
A solar cell in which, a layer obtained by subjecting a sensitizing dye such as a ruthenium dye to monomolecular adsorption on the surface of an inorganic semiconductor such as titania is used as the n layer, and an electrolyte solution is used as the p layer, is called as a dye-sensitized solar cell (so-called a Graetzel cell). Researches on the dye-sensitized solar cell have been energetically conducted since 1991 in view of its high photoelectric conversion efficiency (hereinafter often abbreviated as "conversion efficiency"). However, it has defects that leakage occurs after the use for a long period of time due to the use of a solution, or the like. In order to overcome such defects, researches to obtain an all solid-type dye-sensitized solar cell by solidifying an electrolyte solution have been made recently. However, the technology of allowing an organic substance to be perfused to fine pores of porous titania is very difficult, and therefore, a cell which exhibits a high conversion efficiency with a good reproducibility has not yet been completed.
On the other hand, an organic thin film solar cell in which both of the n layer and the p layer are formed from organic thin films has no defect such as leakage of the solution because it is an all solid-type cell. In addition, it can be easily fabricated, uses no ruthenium which is a rare metal, and has other advantages. Therefore, such an organic thin film solar cell has attracted attention and has been energetically studied.
As for organic thin film solar cells, studies have been made in the early stage on a monolayer film formed of a merocyanine dye or the like. It was found that the photoelectric conversion efficiency (hereinafter often abbreviated as the conversion efficiency) increases by using a multilayer film of a p layer/n layer, and thereafter, such a multilayer film has been mainly employed. For example, Patent document 1 discloses an organic thin film solar cell using copper phthalocyanine (CuPc) for the p layer, peryleneimides (PTCBI) for the n layer, and a nitrogen-containing heterocyclic compound (bathocuproin: BCP) for the exciton blocking layer.
Thereafter, it was found that insertion of an i layer (a mixture layer of a p-material and an n-material) between the p layer and the n layer results in improvement in the conversion efficiency. However, the materials used at that time were phthalocyanines, peryleneimides and BCP as ever. Subsequently, it was found that the conversion efficiency is further improved by employing a stack cell structure in which several p/i/n layers are stacked. The materials used at that time were phthalocyanines, fullerene $C_{60}$ and bathocuproin (BCP).
On the other hand, in an organic thin film solar cell using a polymer compound, studies have been mainly made on the so-called bulkhetero structure in which a conductive polymer is used as the p material (a material constituting a p layer) and a fullerene $C_{60}$ compound is used as the n material (a material constituting an n layer). These materials are mixed and the resulting mixture is subjected to a heat treatment to induce the separation of micro layers, whereby the hetero interface of the p material and the n material is increased to improve the conversion efficiency. The materials used mainly in this structure were a soluble polythiophene compound called as P3HT as the p material and a soluble $C_{60}$ compound called as PCBM as the n material.
As mentioned above, in an organic thin film solar cell, the conversion efficiency was improved by optimizing the cell structure and the morphology of the p layer and the n layer. However, no significant change was made for the materials used in an organic thin film solar cell, and phthalocyanine, peryleneimide, $C_{60}$ and bathocuproin (BCP) have still been used. Therefore, development of new materials replacing these materials has been aspired.
Patent document 2 discloses an organic thin film solar cell using a phenanthroline derivative shown in the following formula in the exciton blocking layer. Attention was attracted to the fact that this phenanthroline derivative has an energy gap equivalent to that of BCP, and the phenanthroline derivative has been used in the exciton blocking layer.

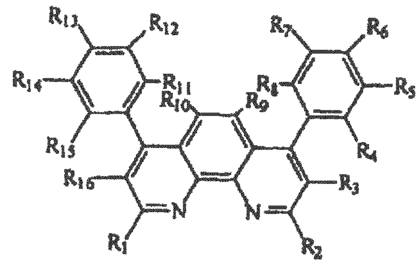

Patent document 3 discloses an electron-transporting stacking film using a perylenetetracarboxylic acid imide represented by the following formula. However, no reference is made on the use thereof in an organic thin film solar cell.

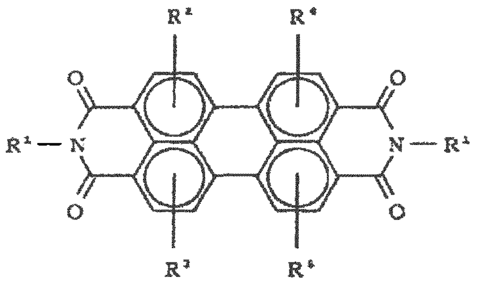

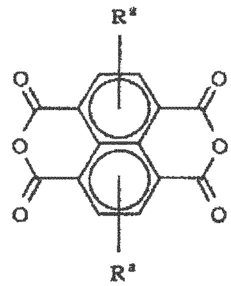

In general, the operation process of an organic solar cell is essentially composed of (1) absorption of light and generation of excitons, (2) diffusion of excitons, (3) separation of electric charges, (4) movement of electric charges and (5) generation of an electromotive force. In general, an organic substance is poor in electron-transporting properties. Further, improvement in efficiency of coupling electrons from an organic layer to a cathode in an organic solar cell has become a subject By solving such a subject, it is possible to obtain an organic thin film solar cell having a high conversion efficiency.

Related Art Document

Patent Documents

Patent Document 1: JP-T-2003-515933
Patent Document 2: JP-T-2008-522413
Patent Document 3: JP-A-H11-95265

Summary of the Invention

An object of the invention is to provide a material for an organic thin film solar cell having high electron-transporting properties and a small energy barrier with electrodes, and show highly efficient photoelectric conversion properties when used in an organic thin film solar cell.
The inventors made extensive studies to attain the above-mentioned subject, and have found that a compound having a phthalic anhydride skeleton, a phthalimide skeleton, a naphthalic anhydride skeleton or a naphthalimide skeleton and a phenanthroline skeleton has a high electron-transporting properties and small energy barrier with electrodes, and hence, when used in an organic thin film solar cell, it shows a highly efficient photoelectric conversion properties. The invention has been made on this finding.
According to the invention, the following compound, electron-transporting material, material for an organic thin film solar cell, organic thin film solar cell, stacked organic thin film solar cell and an apparatus provided with an organic thin film solar cell or a stacked organic thin film solar cell are provided.

1. A compound represented by the following formula (1):

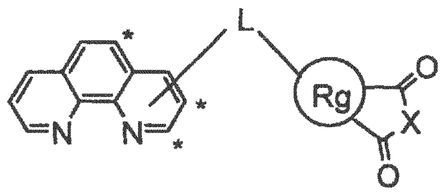

(1)

wherein L is a single bond or a divalent group, and is bonded at any one of the $6^{th}$, $8^{th}$ and the $9^{th}$ positions indicated by * of 1,10-phenanthroline;

Rg is a substituted or unsubstituted benzene ring or a substituted or unsubstituted naphthalene ring; and

X is an oxygen atom or N-$R^4$ (wherein $R^4$ is a hydrogen atom, a halogen atom, a cyano group, a substituted or unsubstituted alkyl group having 1 to 40 carbon atoms, a substituted or unsubstituted alkenyl group having 2 to 40 carbon atoms, a substituted or unsubstituted alkynyl group having 2 to 40 carbon atoms, a substituted or unsubstituted aryl group having 6 to 40 ring carbon atoms, a substituted or unsubstituted heteroaryl group having 5 to 40 ring atoms, a substituted or unsubstituted alkoxy group having 1 to 40 carbon atoms or a substituted or unsubstituted aryloxy group having 6 to 40 ring carbon atoms).

2. The compound according to 1, wherein the compound represented by the formula (1) is a compound represented by the following formula (2) or (3):

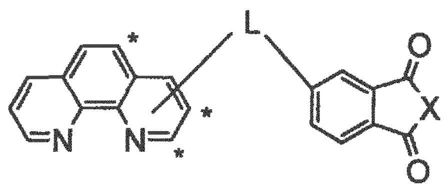

(2)

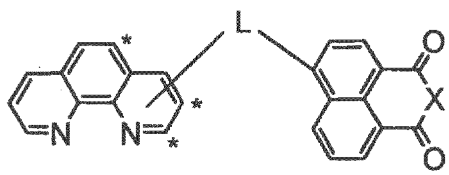

(3)

wherein X is as defined in the formula (1), L is a single bond or a group represented by any of the following formulas (L1) to (L12), and is bonded at any one of the $6^{th}$, $8^{th}$ and the $9^{th}$ positions indicated by * of 1,10-phenanthroline;

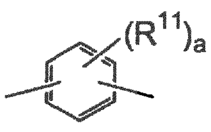

(L1)

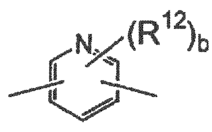

(L2)

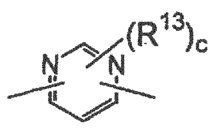

(L3)

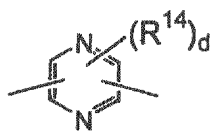

(L4)

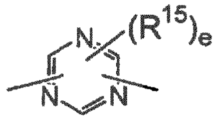

(L5)

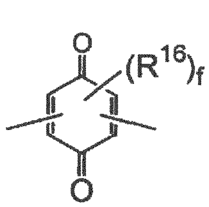

(L6)

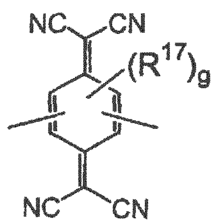

(L7)

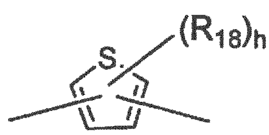

(L8)

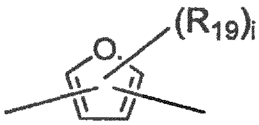
(L9)

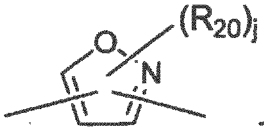
(L10)

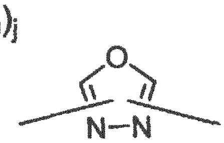
(L11)

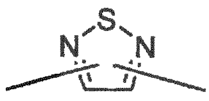
(L12)

wherein $R^{11}$ to $R^{20}$ are independently a halogen atom, a cyano group, a substituted or unsubstituted alkyl group having 1 to 40 carbon atoms, a substituted or unsubstituted alkenyl group having 2 to 40 carbon atoms, a substituted or unsubstituted alkynyl group having 2 to 40 carbon atoms, a substituted or unsubstituted aryl group having 6 to 40 ring carbon atoms, a substituted or unsubstituted heteroaryl group having 5 to 40 ring atoms, a substituted or unsubstituted alkoxy group having 1 to 40 carbon atoms, a substituted or unsubstituted aryloxy group having 6 to 40 ring carbon atoms, a substituted or unsubstituted arylamino group having 6 to 40 ring carbon atoms or a substituted or unsubstituted alkylamino group having 1 to 40 carbon atoms;

a is an integer of 0 to 4, b is an integer of 0 to 3, c is an integer of 0 to 2, d is an integer of 0 to 2, e is 0 or 1, f is an integer of 0 to 2, g is an integer of 0 to 2, h is an integer of 0 to 2, i is an integer of 0 to 2 and j is an integer of 0 or 1; and when a to d and f to i are 2 or more, two or more $R^{11}$s to $R^{14}$s and $R^{16}$s to $R^{19}$s may be the same or different

3. An electron-transporting material comprising the compound according to 1 or 2.
4. A material for an organic thin film solar cell comprising the compound according to 1 or 2.
5. An organic thin film solar cell comprising, between an anode and a cathode, an organic layer that comprises the compound according to 1 or 2.
6. The organic thin film solar cell according to 5, wherein the organic layer is a buffer layer nearer to the cathode.
7. A stacked organic thin film solar cell obtained by stacking a plurality of organic solar cell single cells, wherein a buffer layer which is in contact with an intermediate electrode comprises the compound according to 1 or 2.
8. An apparatus comprising with the organic thin film solar cell according to 5 or 6 or the stacked organic thin film solar cell according to 7.

According to the invention, a phenanthroline compound having a novel specific structure which exhibits highly efficient photoelectric conversion properties when used as a material for an organic thin film solar cell can be provided.
According to the invention, a novel electron-transporting material and a material for an organic thin film solar cell can be provided.
According to the invention, an organic thin film solar cell and a stacked organic thin film solar cell capable of obtaining highly efficient photoelectric conversion properties can be provided.

Mode for Carrying out the Invention

The compound of the invention has a structure represented by the following formula (1):

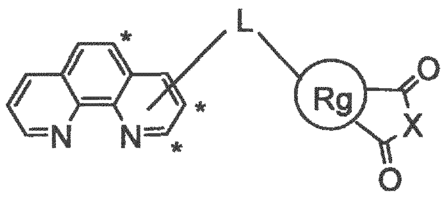
(1)

wherein L is a single bond or a divalent group and is bonded at any one of the 6th, the 8th and the 9th positions indicated by * of 1,10-phenanthroline.
Rg is a substituted or unsubstituted benzene ring or a substituted or unsubstituted naphthalene ring.
X is an oxygen atom or N-$R^4$.
$R^4$ is a hydrogen atom, a halogen atom, a cyano group, a substituted or unsubstituted alkyl group having 1 to 40 carbon atoms, a substituted or unsubstituted alkenyl group having 2 to 40 carbon atoms, a substituted or unsubstituted alkynyl group having 2 to 40 carbon atoms, a substituted or unsubstituted aryl group having 6 to 40 carbon atoms that form a ring (hereinafter referred to as the "ring carbon atoms"), a substituted or unsubstituted heteroaryl group having 5 to 40 atoms that form a ring (hereinafter referred to as the "ring atoms"), a substituted or unsubstituted alkoxy group having 1 to 40 carbon atoms or a substituted or unsubstituted aryloxy group having 6 to 40 ring carbon atoms. $R^4$ is preferably an alkyl group having 1 to 40 carbon atoms.

The compound of the invention which is represented by the formula (1) is characterized by having, within a molecule, a skeleton structure selected from a phthalic anhydride structure, a phthalimide structure, a naphthalic anhydride structure and a naphthalimide structure. The compound of the invention preferably has a phthalimide skeleton and a naphthalimide skeleton.
Due to the presence of these skeleton structures, the compound of the invention has high electron-transporting properties, and has a small energy barrier with an electrode. Therefore, when the compound of the invention is used in an organic thin film solar cell, it is possible to obtain an organic thin film solar cell which exhibits highly efficient photoelectric conversion properties.

In the formula (1), when L is a divalent group, a substituted or unsubstituted arylene group having 6 to 40 ring carbon atoms (specific examples thereof include a group obtained by removing two hydrogen atoms from a benzene ring or the like), a substituted or unsubstituted heteroarylene group having 5 to 40 ring atoms (specific examples thereof include a group obtained by removing two hydrogen atoms from a pyridine ring, a pyrimidine ring, a pyrazine ring, a triazine ring, a thiophene ring, a furan ring, an isoxazole ring, an oxadiazole ring and a thiazole ring or the like), a compound having a quinoid structure (specific examples thereof include a group obtained by removing two hydrogen atoms from a benzoquinone skeleton, a tetracyanoquinodimethane skeleton or the like) can preferably be given.
As for the compound of the invention, due to the presence of the above-mentioned cross-linking group L, charge transporting properties are improved.
It is further preferred that L be a single bond or an arylene group. If L is a single bond, by directly linking phthalimide having excellent electron-transporting properties which can enhance Jsc (short-circuit current density) with a phenanthroline skeleton having a high FF (shape factor), both properties are satisfied, and hence, conversion efficiency can preferably be improved. It is preferred that L be an arylene group such as a phenylene group since electron-transporting properties are increased due to the expansion of the π-electron conjugated system. As the arylene group, a phenylene group is preferable.

It is preferred that the compound of the invention represented by the formula (1) be a compound represented by the following formula (2) or (3).

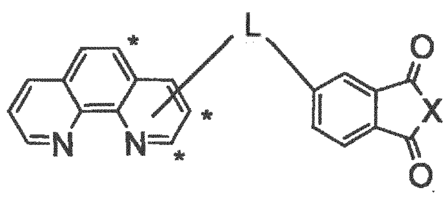

(2)

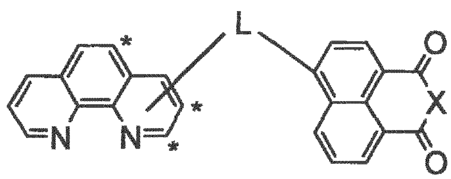

(3)

wherein X is the same as those in the formula (1), L is a single bond or any one of groups represented by the following formulas (L1) to (L12), and bonded at any one of the 6th, the 8th and the 9th positions indicated by * of 1,10-phenanthroline.

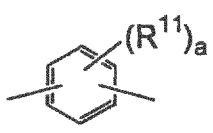

(L1)

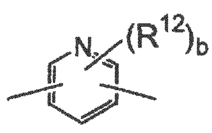

(L2)

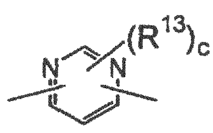

(L3)

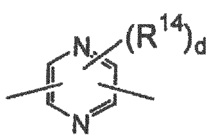

(L4)

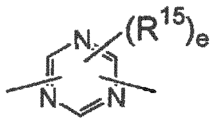 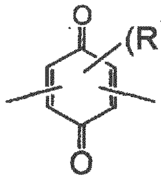 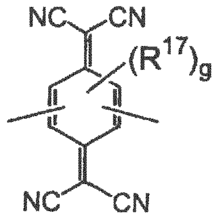 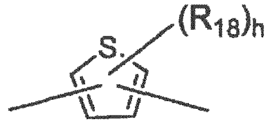

(L 5) (L 6) (L 7) (L 8)

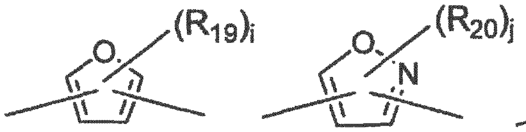 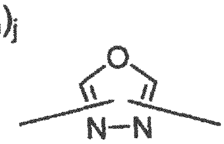 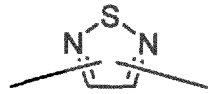

(L 9) (L 1 0) (L 1 1) (L 1 2)

In the formula, $R^{11}$ to $R^{20}$ are independently a halogen atom, a cyano group, a substituted or unsubstituted alkyl group having 1 to 40 carbon atoms, a substituted or unsubstituted alkenyl group having 2 to 40 carbon atoms, a substituted or unsubstituted alkynyl group having 2 to 40 carbon atoms, a substituted or unsubstituted aryl group having 6 to 40 ring carbon atoms, a substituted or unsubstituted heteroaryl group having 5 to 40 ring atoms, a substituted or unsubstituted alkoxy group having 1 to 40 carbon atoms, a substituted or unsubstituted aryloxy group having 6 to 40 ring carbon atoms, a substituted or unsubstituted arylamino group having 6 to 40 ring carbon atoms, or a substituted or unsubstituted alkylamino group having 1 to 40 carbon atoms.

a is an integer of 0 to 4, b is an integer of 0 to 3, c is an integer of 0 to 2, d is an integer of 0 to 2, e is 0 or 1, f is an integer of 0 to 2, g is an integer of 0 to 2, h is an integer of 0 to 2, i is an integer of 0 to 2 and j is an integer of 0 or 1. When a to d and f to i are each 2 or more, two or more $R^{11}$s to $R^{14}$s and $R^{16}$s to $R^{19}$s may independently the same or different.

a to j are preferably 0.

The substituted or unsubstituted alkyl group having 1 to 40 carbon groups represented by $R^4$ and $R^{11}$ to $R^{20}$ may be linear, branched or cyclic. Specific examples thereof include a methyl group, an ethyl group, a 1-propyl group, a 2-propyl group, a 1-butyl group, a 2-butyl group, a sec-butyl group, a tert-butyl group, various pentyl groups, various hexyl groups, various octyl groups, various decyl groups, various dodecyl groups, a 2-ethylhexyl group, a 3,7-dimethyloctyl group, a cyclopropyl group, a cyclopentyl group, a cyclohexyl group, a 1-adamantyl group, a 2-adamantyl group, a norbornyl group, a trifluoromethyl group, a trichloromethyl group, a benzyl group, an $\alpha,\alpha$-dimethylbenzyl group, a 2-phenylethyl group and a 1-phenylethyl group. Of these, in respect of easiness in obtaining raw materials, or for other reasons, a substituted or unsubstituted alkyl group having 1 to 20 carbon atoms are preferable, with a methyl group, an ethyl group, a propyl group, an isopropyl group, a tert-butyl group and a cyclohexyl group being further preferable.

A substituted or unsubstituted alkyl group having 1 to 20 carbon atoms is preferable. Presence of an alkyl group in this range is preferable, since when an organic thin film solar cell is produced by the wet method, solubility in a solvent is increased and coating properties are improved.

As the substituent for the above-mentioned alkyl group, a halogen atom such as a fluorine atom, a chlorine atom, a bromine atom and an iodine atom, and an aryl group such as a phenyl group can be given. The aryl group may be further substituted by an alkyl group having 1 to 5 carbon atoms such as a methyl group, an ethyl group and a propyl group.

The substituted or unsubstituted alkenyl group having 2 to 40 carbon atoms represented by $R^4$ and $R^{11}$ to $R^{20}$ may be linear, branched or cyclic. Specific examples thereof include a vinyl group, a propenyl group, a butenyl group, an oleyl group, an eicosapentaenyl group, a docosahexaenyl group, a styryl group, a 2,2-diphenylvinyl group, a 1,2,2-triphenylvinyl group and a 2-phenyl-2-propenyl group. Of these, a substituted or unsubstituted alkenyl group having 2 to 20 carbon atoms is preferable in respect of easiness in obtaining raw materials, or for other reasons. A vinyl group, a styryl group, a 2,2-diphenylvinyl group or the like are further preferable.

As the substituent for the above-mentioned alkenyl group, an aryl group such as a phenyl group can be given, for example. The aryl group may further be substituted by an alkyl group having 1 to 5 carbon atoms such as a methyl group, an ethyl group and a propyl group.

The substituted or unsubstituted alkynyl group having 2 to 40 carbon atoms represented by $R^4$ and $R^{11}$ to $R^{20}$ may be linear, branched or cyclic. Specific examples thereof include an ethenyl group, a propynyl group and a 2-phenylethenyl group. Of these, a substituted or unsubstituted alkynyl group having 2 to 20 carbon atoms is preferable in respect of easiness in obtaining raw materials, or for other reasons. An ethenyl group and a 2-phenylethenyl group

are further preferable.

As the substituent for the above-mentioned alkynyl group, an aryl group such as a phenyl group can be given, for example. The aryl group may further be substituted by an alkyl group having 1 to 5 carbon atoms such as a methyl group, an ethyl group and a propyl group.

Specific examples of the substituted or unsubstituted aryl group having 6 to 40 carbon atoms represented by $R^4$ and $R^{11}$ to $R^{20}$ include a phenyl group, a 2-tolyl group, a 4-tolyl group, a 4-trifluoromethylphenyl group, a 4-methoxyphenyl group, a 4-cyanophenyl group, a 2-biphenylyl group, a 3-biphenylyl group, a 4-biphenylyl group, a terphenylyl group, a 3,5-diphenylphenyl group, a 3,4-diphenylphenyl group, a pentaphenyphenyl group, a 4-(2,2-diphenylvinyl) phenyl group, a 4-(1,2,2-triphenylvinyl)phenyl group, a fluorenyl group, a 1-naphthyl group, a 2-naphthyl group, a 9-anthryl group, a 2-anthryl group, a 9-phenanthryl group, a 1-pyrenyl group, a chrycenyl group, a naphthacenyl group, and a coronenyl group. Of these, a substituted or unsubstituted aryl group having 6 to 30 ring carbon atoms is preferable in respect of easiness in obtaining raw materials, or for other reasons. A phenyl group, a 4-biphenyl group, a 1-naphthyl group, a 2-naphthyl group, a 9-phenanthryl group or the like are further preferable.

As the substituent for the above-mentioned aryl group, an aryl group such as a phenyl group can be given, for example. The aryl group may further be substituted by an alkyl group having 1 to 5 carbon atoms such as a methyl group, an ethyl group and a propyl group.

As for the substituted or unsubstituted heteroaryl group having 5 to 40 ring atoms represented by $R^4$ and $R^{11}$ to $R^{20}$, in the case of a nitrogen-containing azole-based heterocyclic ring, the heterocyclic ring can be bonded through not only carbon but also through nitrogen. Specific examples thereof include furanyl, thiophenyl, pyrrolyl, imidazolyl, benzimidazolyl, pyrazolyl, benzpyrazolyl, triazolyl, oxadiazolyl, pyridyl, pyrazyl, triazyl, quinoly, benzofuranyl, dibenzofuranyl, benzothiophenyl, dibenzothiophenyl and carbazolyl. Of these, a substituted or unsubstituted heteroaryl group having 5 to 20 ring atoms is preferable in respect of easiness in obtaining raw materials, or for other reasons. A furanyl group, a thiophenyl group, a pyridyl group and a carbazolyl group are further preferable.

As the substituent for the above-mentioned heteroaryl group, an aryl group such as a phenyl group can be given. The aryl group may be further substituted by an alkyl group having 1 to 5 carbon atoms such as a methyl group, an ethyl group and a propyl group.

The substituted or unsubstituted alkoxy group having 1 to 40 carbon atoms represented by $R^4$ and $R^{11}$ to $R^{20}$ may be linear, branched or cyclic. Specific examples thereof include a methoxy group, an ethoxy group, a 1-propyloxy group, a 2-propyloxy group, a 1-butyloxy group, a 2-butyloxy group, a sec-butyloxy group, a tert-butyloxy group, various pentyloxy groups, various hexyloxy groups, various octyloxy groups, various decyloxy groups, various dodecyloxy groups, a 2-ethylhexyloxy group, a 3,7-dimethyloctyloxy group, a cyclopropyloxy group, a cyclopentyloxy group, a cyclohexyloxy group, a 1-adamantyloxy group, a 2-adamantyloxy group, a norbomyloxy group, a trifluoromethoxy group, a benzyloxy group, an $\alpha,\alpha$-dimethylbenzyloxy group, a 2-phenylethoxy group and a 1-phenylethoxy group. Of these, a substituted or unsubstitued alkoxy group having 1 to 20 carbon atoms is preferable in respect of easiness in obtaining raw materials, or for other reasons. A methoxy group, an ethoxy group, a tert-butyloxy group and the like are further preferable.

As the substituent for the above-mentioned alkoxy group, a halogen atom such as a fluorine atom, a chloride atom, a bromine atom and an iodine atom and an aryl group such as a phenyl group are given. The aryl group may further be substituted by an alkyl group having 1 to 5 carbon atoms such as a methyl group, an ethyl group and a propyl group.

Specific examples of the substituted or unsubstituted aryloxy group having 6 to 40 ring carbon atoms represented by $R^4$ and $R^{11}$ to $R^{20}$ include a substituent in which the aryl group is bonded to a nitrogen atom or a group represented by L1 to L12 through oxygen. Of these, a phenoxy group, a naphthoxy group, a phenanthryloxy group or the like are preferable in respect of easiness in obtaining raw materials, or for other reasons.

As the substituent of the aryloxy group, an aryl group such as a phenyl group can be given. The aryl group may be further substituted by an alkyl group having 1 to 5 carbon atoms such as a methyl group, an ethyl group and a propyl group.

As for the substituted or unsubstituted arylamino group having 6 to 40 ring carbon atoms represented by $R^{11}$ to $R^{20}$, it suffices that at least one of the substituents bonding to the amino group be a substituted or unsubstituted aryl group. Specific examples thereof include a phenylamino group, a methylphenylamino group, a diphenylamino group, a di-p-tolylamino group, a di-m-tolylamino group, a phenyl-m-tolylamino group, a phenyl-1-naphthylamino group, a phenyl-2-naphthylamino group, a phenyl(sec-butylphenyl)amino group, a phenyl-t-butylamino group, a bis(4-methoxyphenyl) amino group and a phenyl-4-carbozolylphenylamino group. Of these, a diphenylamino group, a ditolylamino group and a bis(4-methoxyphenyl)amino group or the like are preferable in respect of easiness in obtaining raw materials, or for other reasons..

As the substituent for the arylamino group, an aryl group such as a phenyl group can be given. The aryl group may further be substituted by an alkyl group having 1 to 5 carbon atoms such as a methyl group, an ethyl group and a propyl group.

As for the substituted or unsubstituted alkylamino group having 1 to 40 carbon atoms represented by $R^{11}$ to $R^{20}$, it suffices that at least one of the substituents bonding to the nitrogen atom of the amino group be a substituted or unsubstituted alkyl group. Examples of which include an alkyl group, a hydroxyalkyl group or an alkoxyalkyl group. When

two or more substituted or unsubstituted alkyl groups are bonded to an amino group, these groups may be the same or different and may be bonded with each other to form a ring. Specific examples thereof include a methylamino group, a dimethylamino group, a methylethylamino group, a diethylamino group, a bis(2-hydroxyethyl)amino group, a bis(2-methoxyethyl)amino group, a piperidino group and a morpholino group. Of these, a dimethylamino group, a diethylamino group, a piperidino group or the like are preferable in respect of easiness in obtaining raw materials, or for other reasons. As the substituent for the alkylamino group, an aryl group such as a phenyl group can be given, for example. The aryl group may further be substituted by an alkyl group having 1 to 5 carbon atoms such as a methyl group, an ethyl group and a propyl group.

Rg in the formula (1) is a substituted or unsubstituted benzene ring or a substituted or unsubstituted naphthalene ring. As the examples of the substituent of Rg, the same groups as $R^{11}$ to $R^{20}$ as mentioned above can be given. Specific examples include a halogen atom, a cyano group, a substituted or unsubstituted alkyl group having 1 to 40 carbon atoms, a substituted or unsubstituted alkenyl group having 2 to 40 carbon atoms, a substituted or unsubstituted alkynyl group having 2 to 40 carbon atoms, a substituted or unsubstituted aryl group having 6 to 40 ring carbon atoms, a substituted or unsubstituted heteroaryl group having 5 to 40 ring atoms, a substituted or unsubstituted alkoxy group having 1 to 40 carbon atoms, a substituted or unsubstituted aryloxy group having 6 to 40 ring carbon atoms, a substituted or unsubstituted arylamino group having 6 to 40 ring carbon atoms or a substituted or unsubstituted alkylamino group having 1 to 40 carbon atoms.

Of the compounds of the invention, as the preferable compound having a phthalimide skeleton represented by the above formula (2), a compound having the following structure can be given, for example.

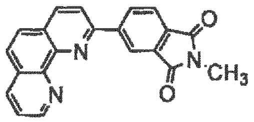
(2-A)

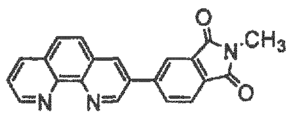
(2-B)

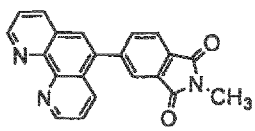
(2-C)

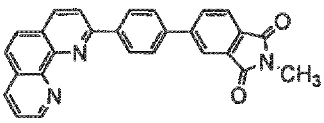
(2-D)

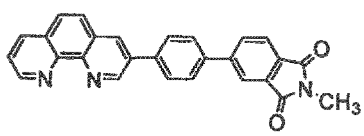
(2-E)

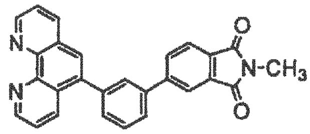
(2-F)

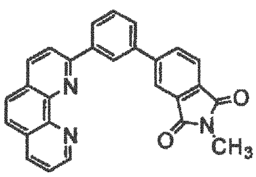
(2-G)

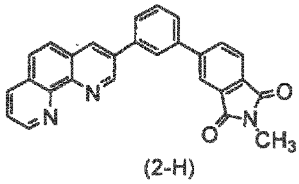
(2-H)

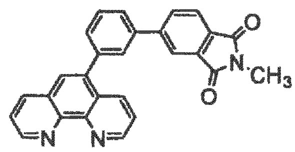
(2-I)

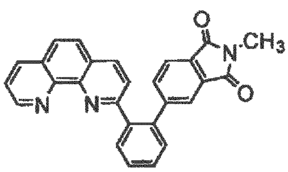
(2-J)

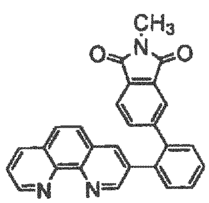
(2-K)

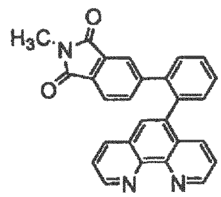
(2-L)

Of the compounds of the invention, as the further preferable compound represented by the above formula (2) having a phthalimide skeleton, a compound having the following structure can be given, for example.

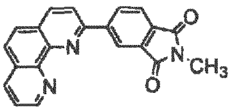

2-A

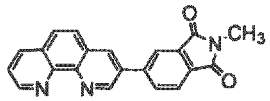

2-B

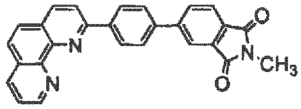

2-D

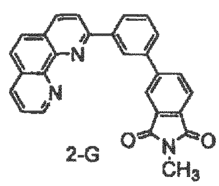

2-G

Of the compounds of the invention, as the preferable compound represented by the formula (3) having a naphthalimide skeleton, a compound having a N-methylnaphthalimide skeleton which bonds at the $4^{th}$ position to a bonding group or a phenanthroline skeleton instead of the N-methylphthalimide skeleton in (2-A) to (2-L) given above can be given.

Various methods can be given as the method for synthesizing the compound of the invention represented by the formula (2). Of these methods, a cross coupling reaction using a metal catalyst, the representative example of which is a palladium catalyst, can be used. A synthesis route using a cross coupling reaction is preferable in respect of easiness in obtaining raw materials, moderate reaction conditions, obtaining an intended product at a high yield or for other reasons. One example of the synthesis route in which L is substituted at the $8^{th}$ position of 1,10-phenanthroline in the above formula (2) is shown below.

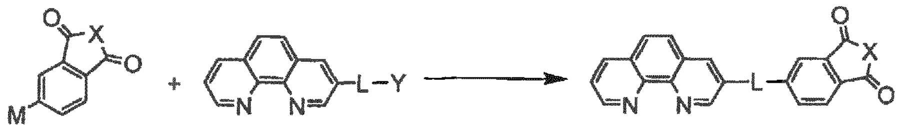

(Reaction formula 1)

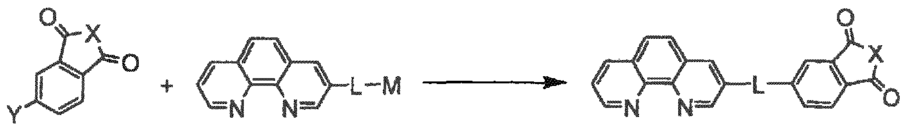

(Reaction formula 2)

Here, Y is a halogen atom such as chlorine, bromine and iodine or a pseudo halogen group such as a trifluoromethanesulfonyloxy group, a nonafluorobutanesulfonyloxy group, a methansulfonyloxy group and a p-toluenesulfonyloxy group.

M is a boronic acid such as $-B(OH)_2$, a pinacolatoboryl group, a catecholboryl group or the like and its ester reagent, an organic tin reagent such as $Sn(Bu)_4$, a typical metal element group represented by an organic silicon reagent such as $-Si(OH)_3$. The typical metal element group may be bonded to phthalic anhydride (reaction formula 1) and may be bonded to a phenanthroline compound through a cross linking group L (reaction formula 2).

As for the conditions under which these typical organic metal reagents are reacted with the bonding unit having these pseudohalogen substituents, a reaction named after a chemist such as a Kumada-Tamao coupling reaction (a Grignard reagent), a Negishi coupling reaction (an organic zinc reagent), a Suzuki-Miyaura coupling reaction (a boron reagent), a Kosugi-Uda-Stille coupling reaction (an organic tin reagent), and a Hiyama coupling reaction (organic silicon reagent) can be given.

Further, if a naphthalic acid anhydride derivative is used instead of a phthalic acid anhydride derivative, a compound represented by the above formula (3) can be produced by the similar synthesis route as shown below.

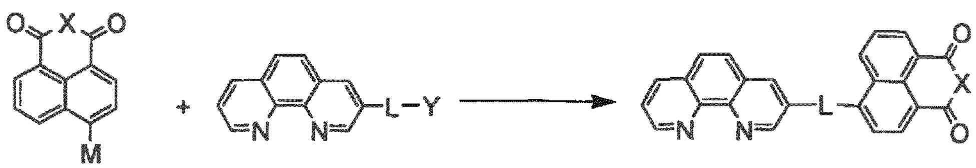

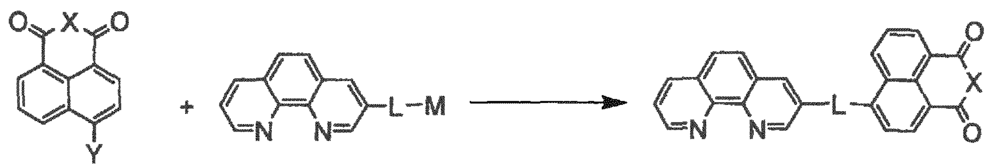

(Y and M in the above formulas are the same as those mentioned above in which phthalic anhydride is used)

As the metal catalyst which can be used in the above-mentioned reaction, a divalent palladium such as palladium chloride, palladium acetate and dichlorobis(triphenylphosphine)palladium, a 0-valent palladium such as tetrakis(triphenylphosphine)palladium and tris(dibenzylideneacetone)dipalladium, a divalent nickel such as nickel chloride, dichlorobis (triphenylphosphine)nickel and dichloro(1,3-bisdiphenylphosphinopropane)nickel, a 0-valent nickel such as tetrakis (triphenylphosphine)nickel, tetracarbonyl nickel, bis(cyclooctadiene)nickel can be given.

Further, a ligand may be added to these metal catalysts. As the ligand which can be used, pyridines such as 2,2'-bipyridine and 1,10-phenanthroline, monodentate phosphines such as triphenylphosphine, tri(o-tolyl)phosphine, tri (2-furyl)phosphine, tricyclohexylphosphine, tri(t-butyl)phosphine, 2-di-t-butylphoshinobiphenyl (JohnPhos), 2-di-t-butylphosphino-2'-dimethylaminobiphenyl (DavePhos), 2-dicyclohexylphosphino-2',4',6'-trisopropyl-1,1'-biphenyl (XPhos), 2-dicyclohexylphosphino-2',6'-dimethoxybiphenyl (SPphos), and bidentate phosphines such as 1,2-bis(diphenylphosphino)ethane (DPPE), 1,3-bis(diphenylphosphino)propane (DPPP), 2,2'-bis(diphenylphosphino)-1,1'-binaphthyl (BINAP), 1,1'-bis(diphenylphosphino)ferrocene (DPPF), 4,5-bis(diphenylphosphino)-9,9-dimethylxanthene (Xantphos) or the like can be given. Of these, in respect of attaining a high yield and moderate reaction conditions, phosphines are preferable.

The compound of the invention is effective as the electron-transporting material and the material for an organic thin film solar cell since it has high electron-transporting properties and has a small energy barrier with electrodes.
The electron-transporting material and the material for an organic thin film solar cell of the invention are characterized by comprising the compound of the invention.
The electron-transporting material and the material for an organic thin film solar cell of the invention may comprise the compound of the invention singly or may comprise the compound of the invention and other components in combination.
The elements of the organic thin film solar cell comprising the material for the organic thin film solar cell of the invention may comprise the material of the invention singly or a mixture of the material of the invention and other components.
An organic thin film solar cell using the material of the invention exhibits highly efficient conversion characteristics.

The organic thin film solar cell of the invention is characterized in that an organic layer comprising the compound of the invention is disposed between a pair of electrodes. Here, the "organic layer" means a layer comprising an organic compound in a solar cell.
In the organic thin film solar cell of the invention, it suffices that at least one layer of one or more organic layers constituting a cell comprise the compound of the invention. Further, an element which comprises the compound of the invention may comprise other components in combination. As for an element which does not comprise the compound of the invention or a mixed material, a known element or material used in an organic thin film solar cell can be used.
It is preferred that the organic layer comprising the compound of the invention be a buffer layer nearer to the cathode.

No particular restrictions are imposed on the cell structure of the organic thin film solar cell of the invention as long as it is a structure in which the organic layer comprising the compound of the invention is contained between a pair of electrodes. Specifically, structures in which the following constitutional elements are provided on a stable insulating substrate can be given.

(1) Lower etectrode/p layer/n layer/upper electrode
(2) Lower electrode/buffer etectrode/p layer/n layer/upper electrode
(3) Lower electrode/p layer/n layer/buffer layer/upper electrode
(4) Lower electrode/buffer layer/p layer/n layer/buffer layer/upper electrode
(5) Lower electrode/buffer layer/p layer/i layer/(or a mixture layer of a p material and an n material)/n layer/buffer layer/upper electrode
(6) Lower etectrode/p layer/n layer/buffer layer/intermediate layer/buffer layer/p layer/n layer/buffer layer/upper electrode
(7) Lower electrode/buffer layer/p layer/n layer/buffer layer/intermediate electrode/buffer layer/p layer/n layer/buffer layer/upper electrode
(8) Lower electrode/buffer layer/p layer/i layer (or a mixture layer of a p material and an n material)/n layer/buffer layer/intermediate electrode/buffer layer/p layer/i layer/(or a mixture layer of a p material and an n material)/n layer/buffer layer/upper electrode

Of the above-mentioned device structures, it is preferred that the compound of the invention be used in a buffer layer in the structures (2) to (8), in particular, a buffer layer nearer to the cathode or a buffer layer which is in contact with an intermediate electrode. It is preferable to use the compound of the invention in a buffer layer of the device structures (3) to (8), in particular, a buffer layer nearer to the cathode or a buffer layer nearer to the intermediate electrode. Hereinbelow, each component will be briefly explained.

1. Lower electrode and upper electrode

No particular restrictions are imposed on the material for the lower electrode and the upper electrode, and a known conductive material can be used. For example, as the electrode connecting with the p layer, a metal such as tin-doped indium oxide (ITO), gold (Au), osmium (Os) and palladium (Pd) can be used. As the electrode connecting with the n layer, metals such as silver (Ag), aluminum (Al), indium (In), calcium (Ca), platinum (Pt) and lithium (Li); a binary metal system such as Mg:Ag, Mg:In or Al:Li can be used. As the electrode connecting with the n layer, a metal or metal system having a small function is preferable.

To obtain high efficient photoelectric conversion properties, it is desired that at least one surface of the organic thin film solar cell preferably have sufficient transparency in the solar light spectrum. The transparent electrode can be formed of a known conductive material by deposition, sputtering or the like such that the predetermined light transmittance is ensured. It is preferred that the light transmittance of the electrode on the light-receiving surface be 10% or more. In a preferable configuration in which a pair of electrodes is provided, one of the electrodes contains a metal having a large work function and the other contains a metal having a small work function.

2. p layer, n layer and i layer

No specific restrictions are imposed on the material of the n layer. However, it is preferable to use a compound having a function as an electron acceptor. For example, as organic compounds, fullerene compounds such as $C_{60}$, carbon nanotube, perylene compounds, polycyclic quinones and quinacridone can be given, and as polymers, CN-poly (phenylene-vinylene), MEH-CN-PPV, polymers having a -CN group or a $CF_3$ group and poly(fluorene) compounds can be given. Preferred are materials having a high electron mobility. More preferred are materials having a small electron affinity. Use of such a material having a small electron affinity in combination for the n layer can lead to a sufficient open-circuit voltage.

As inorganic compounds, an inorganic semiconductor compound having n-type characteristics can be given. Specific examples include doped semiconductors and compound semiconductors such as n-Si, GaAs, CdS, PbS, CdSe, InP, $Nb_2O_5$, $WO_3$ and $Fe_2O_3$; and titanium oxides such as titanium dioxide ($TiO_2$), titanium monoxide (TiO) and dititanium trioxide ($Ti_2O_3$); and conductive oxides such as zinc oxide (ZnO) and tin oxide ($SnO_2$). The above-mentioned inorganic semiconductor compounds having an n-type characteristic may be used alone or in combination of two or more. Titanium oxide is preferably used, with titanium dioxide being particularly preferable.

No specific restrictions are imposed on the material of the p layer. However, it is preferable to use a compound having a function as a hole acceptor. For example, as organic compounds, amine compounds represented by N,N'-bis (3-tolyl)-N,N'-diphenylbenzidine (mTPD), N,N'-dinaphthyl-N,N'-diphenylbenzidine (NPD) and 4,4',4"-tris(phenyl-3-tolylamino)triphenylamine (MTDATA); phthalocyanines such as phthalocyanine (Pc), copper phthalocyanine (CuPc), zinc phthalocyanine (ZnPc) and titanylphthalocyanine (TiOPc); and porphyrins represented by octaethylporphyrin (OEP), platinum octaethylporphyrin (PtOEP) and zinc tetraphenylporphyrin (ZnTPP) can be given. As polymer compounds, main chain-type conjugated polymers such as polyhexylthiophene (P3HT) and methoxyethylhexyloxyphenylenevinylene (MEHPPV), and side chain-type polymers represented by polyvinyl carbazole can be given.

When the compound of the invention is used in the i layer, the i layer may be formed by mixing the p layer compound and the n layer compound as mentioned above. However, it is possible to use the compound of the invention singly as the i layer As the p layer and the n layer in such a case, any of the compounds exemplified above can be used.

3. Buffer layer

In general, an organic thin film solar cell has a small total film thickness, and hence, the upper electrode and the lower electrode often short-circuit so that a yield in fabrication of the cells may decrease. Such short-circuit can be avoided by stacking a buffer layer.

Since the compound of the invention has high electron-transporting properties and small energy barrier with electrodes, it is preferred that the compound of the invention be used in the buffer layer, in particular, in the buffer layer nearer to the cathode.

Further, when the organic thin film solar cell of the invention is a stacked solar cell, it is preferred that the compound of the invention be used in the buffer layer which is in contact with the intermediate electrode.

As materials other than the compound of the invention for forming the buffer layer, preferred are compounds having a sufficiently high carrier mobility such that the short-circuit current does not decrease even when the film thickness of the buffer layer increases. Examples of the material for the buffer layer include, as a low molecular compound, aromatic cyclic acid anhydrides represented by NTCDA as shown below, and as a polymer compound, known conductive polymers represented by poly(3,4-ethylenedioxy)thiophene:polystyrene sulfonate (PEDOT:PSS) and polyaniline:camphor sulfonic acid (PANI:CSA) as shown below.

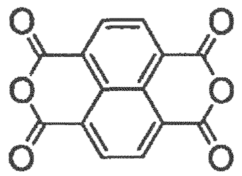

NTCDA

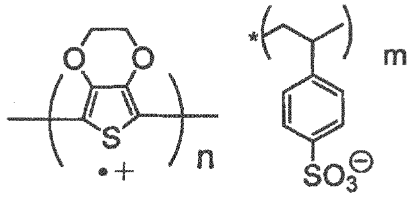

PEDOT : PSS

In addition to the above-mentioned compounds, the inorganic semiconductor compounds exemplified as the materials for the n layer as mentioned above may be used as the materials for the buffer layer. Further, as the CdTe, p-Si, SiC, GaAs, NiO, $WO_3$, $V_2O_5$ or the like p-type inorganic semiconductor compounds can be used.

4. Substrate

As the material for the substrate, materials having mechanical strength, thermal strength and transparency are preferable. Examples of the substrate include glass substrates and transparent resin films. The transparent resin films include films made of polyethylene, ethylene-vinyl acetate copolymer, ethylene-vinyl alcohol copolymer, polypropylene, polystyrene, poly(methyl methacrylate), polyvinylchloride, polyvinylalcohol, polyvinylbutyral, nylon, polyether ether ketone, polysulfone, polyether sulfone, tetrafluoroethylene-perfluoroalkylvinyl ether copolymer, polyvinylfluoride, tetrafluoroethylene-ethylene copolymer, tetrafluoroethylene-hexafluoropropylene copolymer, polychlorotrifluoroethylene, polyvinylidene fluoride, polyester, polycarbonate, polyurethane, polyimide, polyetherimide, polyimide and polypropylene.

5. Stacked organic thin film solar cell

A stacked organic thin film solar cell is called a tandem solar cell, in which at least two power generation layers (organic solar single cell) and at least one intermediate electrode between a pair of electrodes are provided. In a typical structure, a transparent electrode such as ITO is provided on a transparent substrate. A front cell composed of a p layer/i layer/n layer is formed thereon. On an intermediate electrode provided thereon, a back cell composed of a p layer/i layer/n layer is formed, and a cathode is then formed on the back cell. By using such a multi-layer structure, conversion efficiency can be improved.
The same explanation is given on the upper electrode, the lower electrode, the p layer, the n layer, the i layer and the buffer layer constituting the stacked organic thin film solar cell.
It is preferred that the stacked organic thin film solar cell of the invention contain the compound of the invention in the buffer layer which is in contact with the intermediate electrode.

6. Intermediate electrode

By forming an electron-hole recombination zone by the intermediate electrode, it is possible to separate photoelectric conversion units of the stacked device. This layer serves to prevent formation of inverted hetero junction between the n layer of the front photoelectric conversion unit (front cell) and the p layer of the back photoelectric conversion unit (back cell). The layer between each photoelectric conversion unit provides a zone in which electrons injected from the front photoelectric conversion unit and holes injected from the back photoelectric conversion unit are recombined. Efficient recombination of electrons injected from the front photoelectric conversion unit and holes injected from the back photoelectric conversion unit is required when photoinduced current is attempted to be generated in a stacked device.
No particular restrictions are imposed on a material for forming an electron-hole recombination zone by the intermediate electrode. The material for forming the upper electrode and the lower electrode as mentioned above can be used. Preferably, an electron-hole recombination zone by the intermediate electrode contains a thin metal layer.
It is required that the metal layer be sufficiently thin and semi-transparent so that light can reach the back (a plurality of) photoelectric conversion unit. For this purpose, the thickness of the metal layer is preferably smaller than about 20A. It

is particular preferred if the metal film be about 5Å-thick. It is considered that these extremely thin metal films (~5Å) are not continuous films, but rather composed of independent metal nano particles. Surprisingly, although this significantly thin metal layer is not continuous, this is still effective as an electron-hole recombination layer. Examples of preferable metals used in this layer include Ag, Li, LiF, Al, Ti and Sn. Of these metals, Ag is particularly preferable.

7. Method for producing a solar cell

Each layer of the organic thin film solar cell or the stacked organic thin film solar cell of the invention can be formed by dry film-forming methods such as vacuum vapor deposition, sputtering, plasma coating, and ion plating, and wet film-forming methods such as spin coating, dip coating, casting, roll coating, flow coating and inkjet.
The film thickness of each layer is not particularly limited, but the film can be made into an appropriate film thickness. It is generally known that the exciton diffusion length of an organic thin film is short. If the film thickness is too thick, excitons may be deactivated before they reach the hetero interface, so that photoelectric conversion efficiency becomes low. On the other hand, if the film thickness is too thin, generation of pinholes or other problems may occur. As a result, the sufficient diode properties cannot be obtained, resulting in lowering of the conversion efficiency. The appropriate film thickness of each layer is usually in a range of 1 nm to 10 $\mu$m, and further preferably in a range of 5 nm to 0.2 $\mu$m.
In the case of employing the dry film-forming method, known resistance heating methods are preferable. In the case of forming a mixture layer, a film-forming method in which co-deposition is conducted using plural evaporation sources is preferable, for example. Further preferred is to control the substrate temperature during film-formation.
In the case of employing the wet film-forming method, a material for forming each layer is dissolved or dispersed in an appropriate solvent to prepare a luminescent organic solution, and a thin film is formed from the solution. The solvent can be arbitrarily selected. The usable solvent includes halogenated hydrocarbon-based solvents such as dichloromethane, dichloroethane, chloroform, carbon tetrachloride, tetrachloroethane, trichloroethane, chlorobenzene, dichlorobenzene and chlorotoluene; ether-based solvents such as dibutyl ether, tetrahydrofuran, dioxane and anisole; alcohol-based solvents such as methanol, ethanol, propanol, butanol, pentanol, hexanol, cyclohexanol, methyl cellosolve, ethyl cellosolve and ethylene glycol; hydrocarbon-based solvents such as benzene, toluene, xylene, ethylbenzene, hexane, octane, decane and tetralin; and ester-based solvents such as ethyl acetate, butyl acetate and amyl acetate. Of these, the hydrocarbon-based solvents and the ether solvents are preferable. These solvents may be used alone or in a mixture of two or more. Usable solvents are not limited thereto.
In the invention, appropriate resins or additives may be used in any of the organic thin film layers in the organic thin film solar cell in order to improve film-forming properties or for the prevention of generating pinholes of the film, or the like. Usable resins include insulating resins such as polystyrene, polycarbonate, polyarylate, polyester, polyamide, polyurethane, polysulfone, poly(methyl methacrylate), poly(methyl acrylate) and cellulose, and copolymers thereof; photo-conductive resins such as poly-N-vinylcarbazole and polysilane; and conductive resins such as polythiophene and polypyrrole.
The additives include an antioxidant, an ultraviolet absorbent and a plasticizer.
As the apparatus which is provided with the organic thin film solar cell or the stacked organic thin film solar cell of the invention, an integrated module obtained by integrating them can be given. Specifically, application to watches, mobile phones, mobile PCs or the like can be mentioned.

EXAMPLES

The invention will be described in more detail according to the following examples, below. However, the invention will not be restricted by the following examples.

Example 1: Synthesis of a phenanthroline compound (2-A)

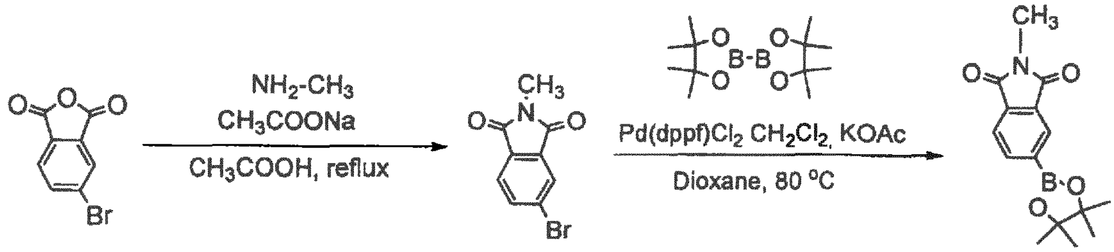


Intermediate A1

Intermediate A2

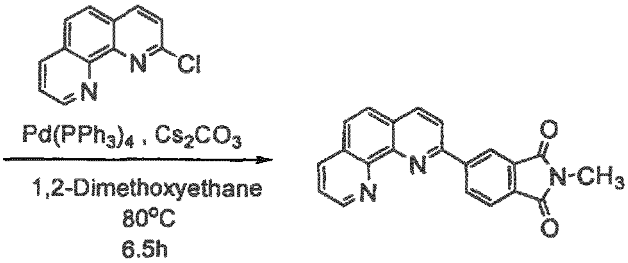


Compound (2-A)

(1) Synthesis of intermediate product A1

In the atmosphere of nitrogen, acetic acid (100 mL) was added to 4-bromo-phthalic anhydride (15g, 66.1 mmol), methylamine (40% in $H_2O$) (8.26 mL, 99.1 mmol) and sodium acetate (8,67g, 106 mmol), followed by reflux while stirring with heating for 6 hours. Water (200 mL) was added to the reaction mixture, and the precipitates were filtrated to obtain white solid (15.2g, 96%).
The nuclear magnetic resonance ($^1$H-NMR) of this solid was measured, and the results are shown below.
$^1$H-NMR (400MHz, $CDCl_3$, TMS) $\delta$: 3.18 (s,3H), 7.71(d, J8.0, 1H), 7.84 (d,J8.0,1H), 7.98(s,1H).

(2) Synthesis of intermediate A2

In the atmosphere of nitrogen, intermediate A1 (10g, 41.7 mmol), bis(pinacolate)diboron (13g, 50.0 mmol), 1,1'-bis(diphenylphosphino)ferrocene]palladium (II)dichloride dichloromethane complex (0.65g, 0.80 mmol), potassium acetate (30g, 305 mmol) and 1,4-dioxane (100 mL) were mixed, followed by stirring at 80°C for 12 hours. Ethyl acetate (300 mL) and water (50 mL) were added to the reaction mixture, an organic phase was collected, dried with magnesium sulfate anhydride, and the solvent was distilled off to obtain a white solid. This white solid was purified by means of column chromatography (silica gel/hexane: ethyl acetate = 3:1) to obtain a white solid (8.0g, 67%).
$^1$H-NMR (400MHz, $CDCl_3$, TMS) $\delta$:1.37 (s,12H), 3.18 (s,3H), 7.82 (d,J8.0,1H), 8.14 (d,J8.0,1H), 8.27 (s, 1H)

(3) Synthesis of a phenanthroline compound (2-A)

In the atmosphere of nitrogen, intermediate A2 (1.6g, 5.6 mmol), 2-chlorophenanthroline (0.8g, 3.7 mmol), tetrakis(triphenylphosphine)palladium (0) (0.1g, 0.11 mmol), cesium carbonate (3.0g, 9.3 mmol) and 1,2-dimethoxyethane (37 mL) were mixed, followed by stirring at 80°C for 6 hours. Water (50 mL) was added to the reaction mixture, and the precipitates were filtered out to obtain a yellow solid (compound (2-A); 0.76g, 60%).
$^1$H-NMR (400MHz, $CDCl_3$, TMS)$\delta$: 3.25 (s,3H), 7.70 (d,J8.0,1H), 8.03 (d,J8.0,1H), 8.18 (d,J8.0,1H), 8.30 (d,J8.0,1 H), 8.41 (d,J8.0,1H), 8.69 (s,1H), 8.83 (d,J8.0,1 H), 9.26 (d,J8.0,1H).

Example 2: Synthesis of a phenanthroline compound (2-B)

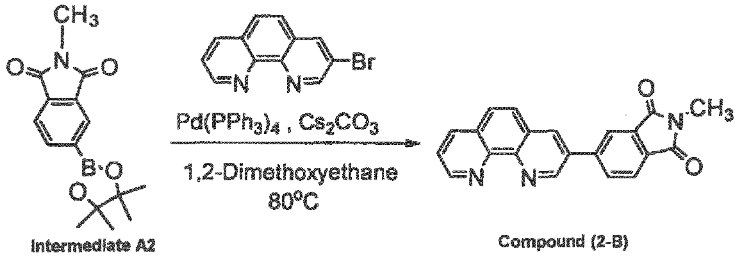


Intermediate A2

Compound (2-B)

In the atmosphere of nitrogen, intermediate A2 (0.33g, 1.15 mmol) synthesized in Example 1 (2), 3-bromophenanthroline (0.2g, 0.77 mmol), cesium carbonate (0.63g, 2.0 mmol), tetrakis(triphenylphosphine)palladium (0) (0.027g, 0.023 mmol) and 1,2-dimethoxyethane (10 mL) were mixed, followed by stirring at 80°C for 6 hours. Water (50 mL) was added to the reaction mixture, and the precipitates were filtered out to obtain a yellow solid (compound (2-B); 0.23g, 90%). $^1$H-NMR (400MHz, $CDCl_3$,TMS)$\delta$: 3.24 (s,3H), 7.69 (d,J8.0,1H), 7.90 (s,1H), 8.03 (d,J8.0,1H), 8.13 (d,J8.0,1H), 8.26-8.31 (m,2H), 8.50 (d,J8.0,1 H), 9.24 (d,J8.0,1 H), 9.46 (d,J8.0,1 H).

Example 3: Synthesis of a phenanthroline compound (2-D)

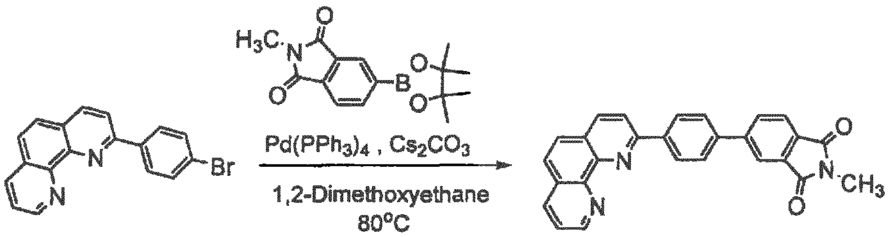


Compound (2-D)

In the atmosphere of nitrogen, intermediate A2 (1.0g, 3.6 mmol) synthesized in Example 1 (2), 4-bromophenyl-2-phenanthroline (1.0g, 2.99 mmol), cesium carbonate (1.75g, 5.4 mmol), tetrakis(triphenylphosphine)palladium (0) (0.17g, 0.15 mmol) and 1,2-dimethoxyethane (30 mL) were mixed, followed by stirring at 80°C for 6 hours. Water (50 mL) was added to the reaction mixture, and the precipitates were filtered out to obtain a yellow solid (compound (2-D); 1.00g, 81 %).
$^1$H-NMR (400MHz,$CDCl_3$, TMS) $\delta$: 3.23 (s,3H), 7.66 (d,J8.0,2H), 7.81-7.87 (m,4H), 7.94 (d,J8.0,2H), 8.02 (d,J8.0,1H), 8.18 (d,J8.0,2H), 8.28 (d,J8.0,1H), 8.36 (d,J8.0,1H), 8.50d,J8.0,2H), 9.26 (d,J8.0,1H).

Example 4: Synthesis of a phenanthroline compound (2-G)

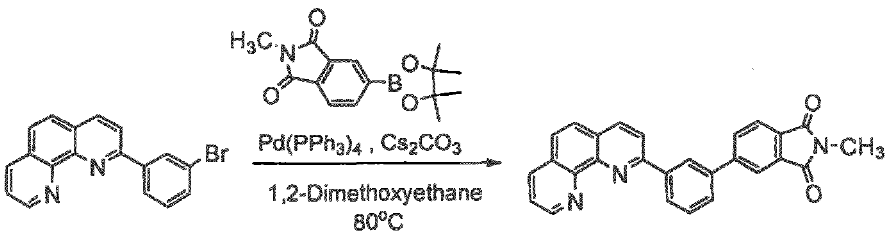


Intermediate A2

Compound (2-G)

In the atmosphere of nitrogen, intermediate A2 (1.0g, 3.6 mmol), 3-bromophenyl-2-phenanthroline (1.0g, 2.99 mmol), cesium carbonate (1.75g, 5.4 mmol), tetrakis(triphenylphosphine)palladium (0) (0.17g, 0.15 mmol) and 1,2-dimethoxyethane (30 mL) were mixed, followed by stirring at 80°C for 6 hours.
Water (50 mL) was added to the reaction mixture, and the precipitates were filtered out to obtain a yellow solid (compound (2-G); 1.06g, 85%).

$^1$H-NMR (400MHz, $CDCl_3$, TMS) δ :3.23 (s,3H), 7.64-7.75 (m,3H), 7.84 (d,J8.0,2H), 7.94 (d,J8.0,1H), 8.08 (d,J8.0,1 H), 8.16-8.20 (m,2H), 8.28 (d,J8.0,1H), 8.36-8.39 (m,2H), 8.58 (s,1H) 9.23 (d,J8.0,1H).

Examples 5 to 8

A glass substrate of 25 mm by 75 mm by 0.7 mm thick with an ITO transparent electrode was subjected to ultrasonic cleaning with isopropyl alcohol for 5 minutes, and cleaned with ultraviolet rays and ozone for 30 minutes. The substrate with transparent electrode lines thus cleaned was mounted on a substrate holder in a vacuum deposition apparatus. CuPc (the structural formula thereof is shown below) was formed into a 30 nm-thick film by the resistance heating deposition at a deposition rate of 1 Å/s so as to cover the surface of the substrate on which the transparent electrode lines were formed as a lower electrode. Subsequently, on this film, $C_{60}$ (the structural formula thereof is shown below) was formed into a 60 nm-thick film by the resistance heating deposition at a deposition rate of 1 Å/s.

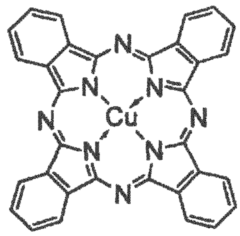

CuPc

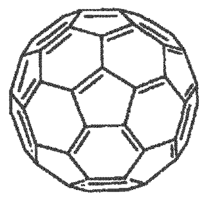

$C_{60}$

On the $C_{60}$ film, any of the compounds (2-A), (2-B), (2-D) and (2-G) synthesized in Examples 1 to 4 was formed into a film of 10 nm by the resistance heating deposition at a deposition rate of 1 Å/s. Finally, metal Al was deposited as the opposing electrode having a film thickness of 80 nm, whereby an organic thin film solar cell was formed. The cell had an area of 0.05 $cm^2$.

The I-V characteristics were determined for the organic thin film solar cell thus fabricated under a condition of AM 1.5 (light intensity: 100 $mW/cm^2$). From the results, the open-circuit voltage (Voc), the short-circuit current density (Jsc) and the fill factor (FF) were obtained, and the photoelectric conversion efficiency (η) was calculated by the following equation: The results are shown in Table 1.

$$\eta\ (\%) = \frac{Voc \times Jsc \times FF}{Pin} \times 100$$

Voc: Open-circuit voltage
Jsc: Short-circuit current density
FF: Fill factor
Pin: Incident light energy.

Comparative Example 1

An organic solar cell was fabricated in the same manner in Example 5, except that the compound (2-A) was not used in Example 5. The I-V characteristics of the thus fabricated organic solar cell were measured under the condition of AM 1.5 (optical intensity: 100 $mW/cm^2$). The open-circuit voltage (Voc), the short-circuit current density (Jsc), the fill factor (FF) and the photoelectric conversion ratio (η) are shown in Table 1.

A device was fabricated in the same manner as in Example 5, except that BCP was used instead of BCP. The I-V characteristics of the thus fabricated organic solar cell were measured under the condition of AM 1.5 (optical intensity: 100 $mW/cm^2$). The open-circuit voltage (Voc), the short-circuit current density (Jsc), the fill factor (FF value) and the photoelectric conversion ratio (η) are shown in Table 1.

Table 1

| | Buffer layer compound | Voc (V) | Jsc (mA/cm$^2$) | FF | η (%) |
|---|---|---|---|---|---|
| Example 5 | Compound 2-A | 0.50 | 7.24 | 0.57 | 2.06 |
| Example 6 | Compound 2-B | 0.51 | 7.30 | 0.56 | 2.08 |
| Example 7 | Compound 2-D | 0.51 | 7.03 | 0.61 | 2.19 |
| Example 8 | Compound 2-G | 0.50 | 6.78 | 0.60 | 2.03 |
| Com. Ex. 1 | None | 0.38 | 1.16 | 0.25 | 0.11 |
| Com. Ex. 2 | BCP | 0.49 | 5.99 | 0.60 | 1.76 |

Example 9

A glass substrate of 25 mm by 75 mm by 0.7 mm thick with an ITO transparent electrode was subjected to ultrasonic cleaning with isopropyl alcohol for 5 minutes, and cleaned with ultraviolet rays and ozone for 30 minutes. The substrate with transparent electrode lines thus cleaned was mounted on a substrate holder in a vacuum deposition apparatus. Compound 1 (the compound disclosed in WO2010/013520A1, the structural formula thereof is shown below) was formed into a 30 nm-thick film by the resistance heating deposition at a deposition rate of 1 Å/s so as to cover the surface of the substrate on which the transparent electrode lines were formed as a lower electrode. Subsequently, on this film, $C_{70}$ (the structural formula thereof is shown below) was formed into a 60 nm-thick film by the resistance heating deposition at a deposition rate of 1 Å/s.

On the $C_{70}$ film, the compound (2-D) synthesized in Example 3 was formed into a 5 nm-thick film by the resistance heating deposition at a deposition rate of 1 Å/s. Subsequently, Ag was formed into a 0.5 nm-thick film by the resistance heating deposition to form an intermediate electrode. Then, $MoO_3$ was formed into a 5 nm-thick film by the resistance heating deposition to form a buffer layer. On the buffer layer, in sequence, compound 1 was formed into a 20 nm-thick film, $C_{70}$ was formed into a 20 nm-thick film and BCP was formed into a 10 nm-thick film. Finally, metal Al was deposited as the counter electrode having a film thickness of 80 nm, whereby an organic thin film solar cell was formed. The cell had an area of 0.05 cm$^2$.

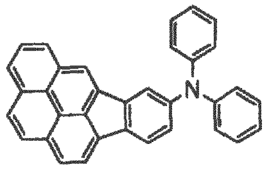

Compound 1

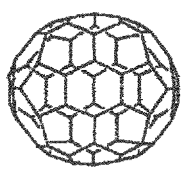

$C_{70}$

The I-V characteristics were determined for the organic solar cell thus fabricated under a condition of AM 1.5 (light intensity (: 100 mW/cm$^2$). The open-circuit voltage (Voc), the short-circuit current density (Jsc), the fill factor (FF) and the photoelectric conversion efficiency (η) are shown in Table 2.

Comparative Example 3

An organic solar cell was fabricated in the same manner as in Example 5, except that the compound (2-D) was not used. The I-V characteristics were determined for the organic solar cell thus fabricated under a condition of AM 1.5 (light intensity: 100 mW/cm$^2$). The open-circuit voltage (Voc), the short-circuit current density (Jsc), the fill factor (FF) and the photoelectric conversion efficiency (η) are shown in Table 2.

Table 2

| | Buffer layer compound | Voc (V) | Jsc (mA/cm$^2$) | FF | η (%) |
|---|---|---|---|---|---|
| Example 9 | Compound 2-D | 1.82 | 3.56 | 0.50 | 3.24 |
| Com. Ex. 3 | None | 1.62 | 1.44 | 0.46 | 1.07 |

As is apparent from Table 1 and Table 2, when the phenanthroline compound of the invention is used in the buffer layer, the conversion efficiency was improved as compared with the organic thin film solar cells of Comparative Examples in which the phenanthroline compound of the invention was not used in the buffer layer. That is, an organic thin film solar cell using the phenanthroline compound of the invention exhibits excellent solar cell properties.

INDUSTRIAL APPLICABILITY

The compound of the invention is useful as the electron-transporting material and the material for an organic thin film solar cell.
According to the invention, it is possible to provide an organic thin film solar cell exhibiting highly efficient photoelectric conversion properties.
Although only some exemplary embodiments and/or examples of this invention have been described in detail above, those skilled in the art will readily appreciate that many modifications are possible in the exemplary embodiments and/or examples without materially departing from the novel teachings and advantages of this invention. Accordingly, all such modifications are intended to be included within the scope of this invention.
The documents described in the specification are incorporated herein by reference in its entirety.

## Claims

1. A compound represented by the following formula (1):

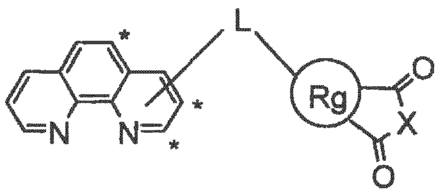

(1)

wherein L is a single bond or a divalent group, and is bonded at any one of the $6^{th}$, $8^{th}$ and the $9^{th}$ positions indicated by * of 1,10-phenanthroline;
Rg is a substituted or unsubstituted benzene ring or a substituted or unsubstituted naphthalene ring; and
X is an oxygen atom or N-$R^4$ (wherein $R^4$ is a hydrogen atom, a halogen atom, a cyano group, a substituted or unsubstituted alkyl group having 1 to 40 carbon atoms, a substituted or unsubstituted alkenyl group having 2 to 40 carbon atoms, a substituted or unsubstituted alkynyl group having 2 to 40 carbon atoms, a substituted or unsubstituted aryl group having 6 to 40 ring carbon atoms, a substituted or unsubstituted heteroaryl group having 5 to 40 ring atoms, a substituted or unsubstituted alkoxy group having 1 to 40 carbon atoms or a substituted or unsubstituted aryloxy group having 6 to 40 ring carbon atoms).

2. The compound according to claim 1, wherein the compound represented by the formula (1) is a compound represented by the following formula (2) or (3):

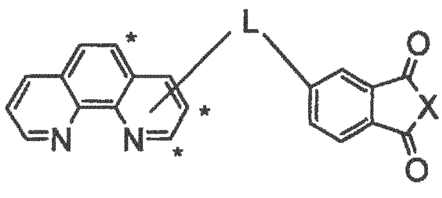

(2)

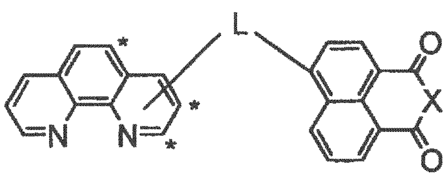

(3)

wherein X is as defined in the formula (1), L is a single bond or a group represented by any of the following formulas (L1) to (L12), and is bonded at any one of the $6^{th}$, $8^{th}$ and the $9^{th}$ positions indicated by * of 1,10-phenanthroline;

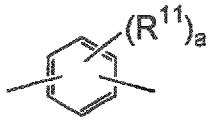
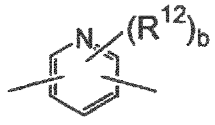
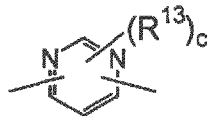
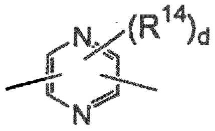

(L1) (L2) (L3) (L4)

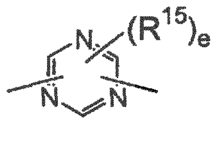
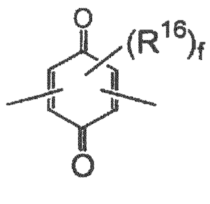
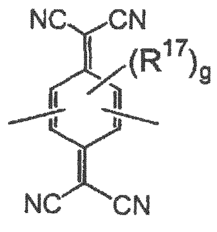
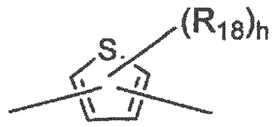

(L5) (L6) (L7) (L8)

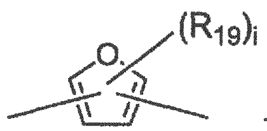
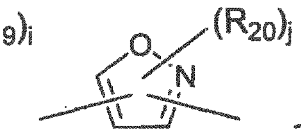
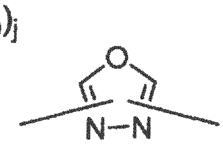
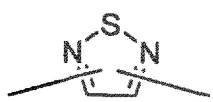

(L9) (L10) (L11) (L12)

wherein $R^{11}$ to $R^{20}$ are independently a halogen atom, a cyano group, a substituted or unsubstituted alkyl group having 1 to 40 carbon atoms, a substituted or unsubstituted alkenyl group having 2 to 40 carbon atoms, a substituted or unsubstituted alkynyl group having 2 to 40 carbon atoms, a substituted or unsubstituted aryl group having 6 to 40 ring carbon atoms, a substituted or unsubstituted heteroaryl group having 5 to 40 ring atoms, a substituted or unsubstituted alkoxy group having 1 to 40 carbon atoms, a substituted or unsubstituted aryloxy group having 6 to 40 ring carbon atoms, a substituted or unsubstituted arylamino group having 6 to 40 ring carbon atoms or a substituted or unsubstituted alkylamino group having 1 to 40 carbon atoms;

a is an integer of 0 to 4, b is an integer of 0 to 3, c is an integer of 0 to 2, d is an integer of 0 to 2, e is 0 or 1, f is an integer of 0 to 2, g is an integer of 0 to 2, h is an integer of 0 to 2, i is an integer of 0 to 2 and j is an integer of 0 or 1; and when a to d and f to i are 2 or more, two or more $R^{11}$s to $R^{14}$s and $R^{16}$s to $R^{19}$s may be the same or different.

**3.** An electron-transporting material comprising the compound according to claim 1 or 2.

**4.** A material for an organic thin film solar cell comprising the compound according to claim 1 or 2.

**5.** An organic thin film solar cell comprising, between an anode and a cathode, an organic layer that comprises the compound according to claim 1 or 2.

**6.** The organic thin film solar cell according to claim 5, wherein the organic layer is a buffer layer nearer to the cathode.

**7.** A stacked organic thin film solar cell obtained by stacking a plurality of organic solar cell single cells, wherein a buffer layer which is in contact with an intermediate electrode comprises the compound according to claim 1 or 2.

**8.** An apparatus comprising the organic thin film solar cell according to claim 5 or 6 or the stacked organic thin film solar cell according to claim 7.

**INTERNATIONAL SEARCH REPORT**

International application No. PCT/JP2011/004246

A. CLASSIFICATION OF SUBJECT MATTER
*C07D471/04*(2006.01)i, *H01L51/42*(2006.01)i

According to International Patent Classification (IPC) or to both national classification and IPC

B. FIELDS SEARCHED

Minimum documentation searched (classification system followed by classification symbols)
C07D471/04, H01L51/42

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 2009-517887 A (EASTMAN KODAK CO.), 30 April 2009 (30.04.2009), claims 1, 12 to 13; paragraphs [0011], [0062] & US 2007/122656 A1 & WO 2007/064493 A1 & EP 1955384 A1 & KR 2008072883 A | 1,3<br>2,4-8 |
| A | JP 2005-108720 A (TDK CORP.), 21 April 2005 (21.04.2005), (Family: none) | 1-8 |
| A | JP 2006-505115 A (DU PONT DE NEMOURS & CO. E I), 09 February 2006 (09.02.2006), & WO 2004/005288 A2 & US 2004/068115 A1 & AU 2003249016 A1 & EP 1519935 A2 | 1-8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>04 October, 2011 (04.10.11) | Date of mailing of the international search report<br>18 October, 2011 (18.10.11) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No.
PCT/JP2011/004246

C (Continuation). DOCUMENTS CONSIDERED TO BE RELEVANT

| Category* | Citation of document, with indication, where appropriate, of the relevant passages | Relevant to claim No. |
|---|---|---|
| A | JP 2008-106015 A (Chemipro Kasei Kaisha, Ltd.), 08 May 2008 (08.05.2008), (Family: none) | 1-8 |
| A | JP 2008-522413 A (UNIV PRINCETON), 26 June 2008 (26.06.2008), & US 2006/138453 A1 & WO 2006/086040 A2 & EP 1815544 A2 & AU 2005327083 A1 & KR 2007089956 A & CN 101076904 A | 1-8 |
| A | JP 2005-320288 A (MITSUI CHEM INC.), 17 November 2005 (17.11.2005), (Family: none) | 1-8 |
| A | JP 11-095265 A (Chemipro Kasei Kaisha, Ltd.), 09 April 1999 (09.04.1999), (Family: none) | 1-8 |
| A | Daniel S. Tyson, et al., Inorganic Chemistry, 2001, 40(16), pages 4063 to 4071 | 1-8 |
| A | Gianluca Ambrosi, et al., Inorganic Chemistry, 2007, 46(11), pages 4737 to 4748 | 1-8 |
| P,A | WO 2010/085121 A2 (UNIV KYUNGPOOK NAT IND ACAD COOP), 29 July 2010 (29.07.2010), (Family: none) | 1-8 |
| P,A | JP 2010-254608 A (Idemitsu Kosan Co., Ltd.), 11 November 2010 (11.11.2010), (Family: none) | 1-8 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003515933 T
- JP 2008522413 T
- JP H1195265 A
- WO 2010013520 A1